# EUROPEAN PATENT SPECIFICATION

(11) **EP 4 205 679 B1**
(45) Date of publication and mention of the grant of the patent: **28.01.2026**
(21) Application number: 22217120.9
(22) Date of filing: 29.12.2022
(51) Int. Cl.: A61B 18/14, A61B 5/25

(54) **BASKET CATHETER HAVING ABLATION ELECTRODES AND TEMPERATURE SENSORS**
KORBKATHETER MIT ABLATIONSELEKTRODEN UND TEMPERATURSENSOREN
CATHÉTER À PANIER COMPORTANT DES ÉLECTRODES D'ABLATION ET DES CAPTEURS DE TEMPÉRATURE

(30) Priority: 30.12.2021 US 202117566336
(43) Date of publication of application: 05.07.2023
(73) Proprietor: Biosense Webster (Israel) Ltd., Yokneam, 2066717 (IL)
(72) Inventor: GOVARI, Assaf, Yokneam 2066717 (IL); BEECKLER, Christopher Thomas, Irvine 92618 (US); KEYES, Joseph Thomas, Irvine 92618 (US); LICHTER, Justin George, Irvine 92618 (US); HERRERA, Kevin Justin, Irvine 92618 (US)
(74) Representative: Carpmaels & Ransford LLP

(56) References cited:
- WO-A1-2021/126980
- US-A1- 2013 116 688
- US-A1- 2014 018 788
- US-A1- 2019 069 949
- US-A1- 2021 045 805

## Description

### FIELD OF THE DISCLOSURE

The present disclosure relates generally to medical devices, and particularly to production methods and expandable catheters having ablation electrodes and temperature sensors.

### BACKGROUND OF THE DISCLOSURE

Producing methods and expandable catheters having ablation electrodes and various types of sensing electrodes have been published.

For example, U.S. Patent Application Publication 2020/0337765 describes systems, devices and methods for modulating targeted nerve fibers (e.g., hepatic neuromodulation) or other tissue. The systems may be configured to access tortuous anatomy of or adjacent hepatic vasculature. The systems may be configured to target nerves surrounding (e.g., within adventitia of or within perivascular space of) an artery or other blood vessel, such as the common hepatic artery.

US 2014/0018788 Al provides nethods and endovascular catheters for assessing, and treating patients having sympathetically mediated disease, involving augmented peripheral chemoreflex and heightened sympathetic tone by reducing chemosensor input to the nervous system via transmural carotid body ablation.

WO 2021/126980 A1 provides pulmonary vein isolation catheters and associated devices, systems, and methods. In some embodiments, a pulmonary vein isolation catheter includes a tip section having an expandable portion and a deployment member. The expandable portion includes a plurality of mesh electrode panels that are electrically insulated from one another.

### SUMMARY OF THE INVENTION

The invention is relates to a catheter according to independent claim 1, and a method for producing such a catheter according to claim 9. Advantageous embodiments are defined in the dependent claims.

The present disclosure will be more fully understood from the following detailed description of the examples thereof, taken together with the drawings in which:

### BRIEF DESCRIPTION OF THE DRAWINGS

Fig. 1 is a schematic, pictorial illustration of a catheter-based position-tracking and ablation system, in accordance with an example of the present disclosure;
Fig. 2 is a schematic, pictorial illustration of a distal-end assembly of a basket catheter in an expanded position, in accordance with examples of the present disclosure;
Fig. 3 is a schematic top view of multiple sensing electrodes as produced, and a sectional view of one sensing electrode, in accordance with examples of the present disclosure;
Fig. 4 is a block diagram that schematically illustrates a process flow for producing one of the sensing electrodes shown in Figs 2 and 3, in accordance with examples of the present disclosure;
Fig. 5 is a flow chart that schematically illustrates a method for producing a basket catheter having ablation electrodes and the sensing electrodes shown in Figs. 2-4, in accordance with an example of the present disclosure; and
Fig. 6 is a flow chart that schematically illustrates a method for producing a basket catheter having ablation electrodes and one or more thermocouples, in accordance with an example of the present disclosure.

### DETAILED DESCRIPTION OF EXAMPLES

### OVERVIEW

Examples of the present disclosure that are described hereinbelow provide improved techniques for producing an expandable catheter, such as a basket catheter, having ablation electrodes, electro-anatomical (EA) sensing electrodes and temperature sensors, such as thermocouples (TCs).

In some examples, an ablation catheter comprises a shaft for insertion into a patient heart, and an expandable distal end, in the present example, a basket-shaped distal-end assembly, also referred to herein as a basket. The basket is coupled to the shaft and is configured to be in a collapsed position when moved through the vasculature to or from the heart, and to be expanded to one or more expanded positions, e.g., for sensing electrocardiogram (ECG) signals and/or for applying ablation signals to tissue of the heart.

The basket comprises multiple arms, also referred to herein as splines. In some examples, at least one of, and typically all the splines, comprise a flexible substrate, which is configured to conform to tissue of the heart when being placed in contact therewith, so as to: (i) sense ECG signals in the tissue, and/or (ii) apply radiofrequency (RF) ablation signals (e.g., pulses) to the tissue via the aforementioned ablation electrodes.

In some examples, at least one of and typically each ablation electrode includes a slot, which is configured to contain a temperature sensing device, such as a thermocouple (TC). When a given ablation electrode that contains a given TC is placed in contact with the tissue, the given TC is configured to produce a thermal signal indicative of the sensed temperature of the tissue. Note that the TC is configured to sense the tissue temperature while the ablation electrode is applying ablation signals to the tissue, and/or when the ablation electrodes is in contact with the tissue, but no ablation signal is applied to the tissue.

In some examples, when placed in contact with tissue of the heart, each of the EA sensing electrodes, also referred to herein as a sensing electrode for brevity, is configured to produce a sensing signal indicative of an ECG signal sensed in the tissue. Each spline may comprise one or more sensing electrodes that are coupled to the flexible substrate at selected positions along a longitudinal axis of the spline.

In some examples, each sensing electrode comprises a gold substrate whose surface includes at least first and second sections that do not overlap one another but have a common interface.

In some examples, a first polymer layer, which is formed over the first section of the gold substrate, is configured to electrically isolate between the tissue and the gold substrate. A second polymer layer, which is formed over the second section of the gold substrate, is configured to conduct the ECG signal between the tissue and the gold substrate.

In some examples, a plurality of the sensing electrodes may be produced together and then separated into one or more sensing electrodes intended to be coupled to a respective spline.

In some examples, the gold substrate includes a first surface intended to be coupled to the flexible substrate of the spline, and a second surface having the first and second sections.

In some examples, in the production process flow: (i) the first polymer layer is applied to the entire surface of the gold substrate, (ii) the first polymer layer is removed from the second section (e.g., using a laser that cuts through the first polymer layer) for exposing the second surface of the gold substrate, and (iii) the second polymer layer is applied to the second section of the second surface of the gold substrate.

In some examples, the second outer surface of the second polymer is recessed relative to the first outer surface of the first polymer, so as to protect the second outer surface from being damaged when placed in contact with the tissue.

In other examples, the outer surface of the first and second polymer layers, which are intended to be placed in contact with the tissue, are typically flush with one another. For example, these examples are obtained by having a similar thickness of the first and second polymer layers.

In alternative examples, the outer surface of the second polymer layer may slightly protrude (e.g., about less than 5 µm) toward the tissue relative to the outer surface of the first polymer layer, so as to conduct the ECG signal from the tissue to the gold substrate by having sufficient contact force between the tissue and the outer surface of the second polymer layer. For example, these examples are obtained by having the thickness of the second polymer slightly layer is larger than that of the first polymer layer.

In some examples, at least one of and typically each spline of the basket catheter may have on their surface intended to be placed in contact with the tissue: (i) at first positions, the one or more ablation electrodes and temperature sensors contained within the slots of the ablation electrodes, and (ii) at second positions that are different from the first positions, the one or more sensing electrodes.

The disclosed techniques improve the functionality of expandable catheters, such as basket catheters, for conducting both electro-anatomical mapping and ablation of tissue in question. Moreover, the disclose techniques reduce the need to insert two different catheters (sensing and ablation) into the patient heart, and therefore, reduce the duration of the medical procedure and reduce the cost associated with using two different catheters for performing the sensing and ablation procedures.

### SYSTEM DESCRIPTION

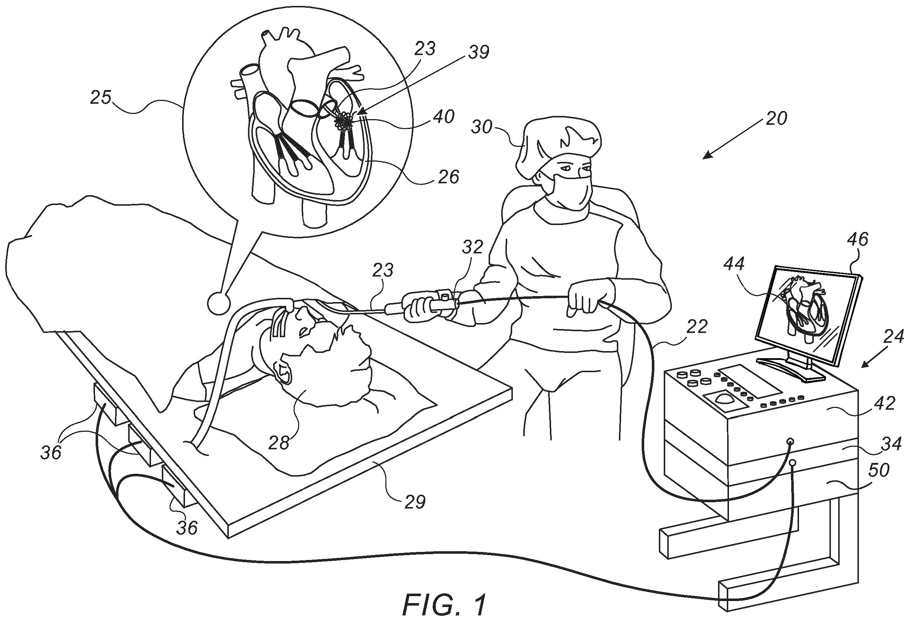

Fig. 1 is a schematic, pictorial illustration of a catheter-based position-tracking and ablation system 20, in accordance with an example of the present disclosure. In some examples, system 20 comprises a catheter 22, in the present example an expandable cardiac catheter having a basket shape, and a control console 24. In the example described herein, catheter 22 may be used for any suitable therapeutic and/or diagnostic purposes, such as but not limited to sensing of electro-anatomical (EA) information in tissue in question and applying ablation signals to tissue of a heart 26. In the context of the present disclosure, the term information refers to the spatial location of the catheter distal end, and an electrocardiogram (ECG) signal sensed by electrodes of catheter 22.

In some examples, console 24 comprises a processor 42, typically a general-purpose computer, with suitable front end and interface circuits for receiving signals from catheter 22 and for controlling other components of system 20 described herein. Processor 42 may be programmed in software to carry out the functions that are used by the system and is configured to store data for the software in a memory 50. The software may be downloaded to console 24 in electronic form, over a network, for example, or it may be provided on non-transitory tangible media, such as optical, magnetic or electronic memory media. Alternatively, some or all of the functions of processor 42 may be carried out using an application-specific integrated circuit (ASIC) or any suitable type of programmable digital hardware components.

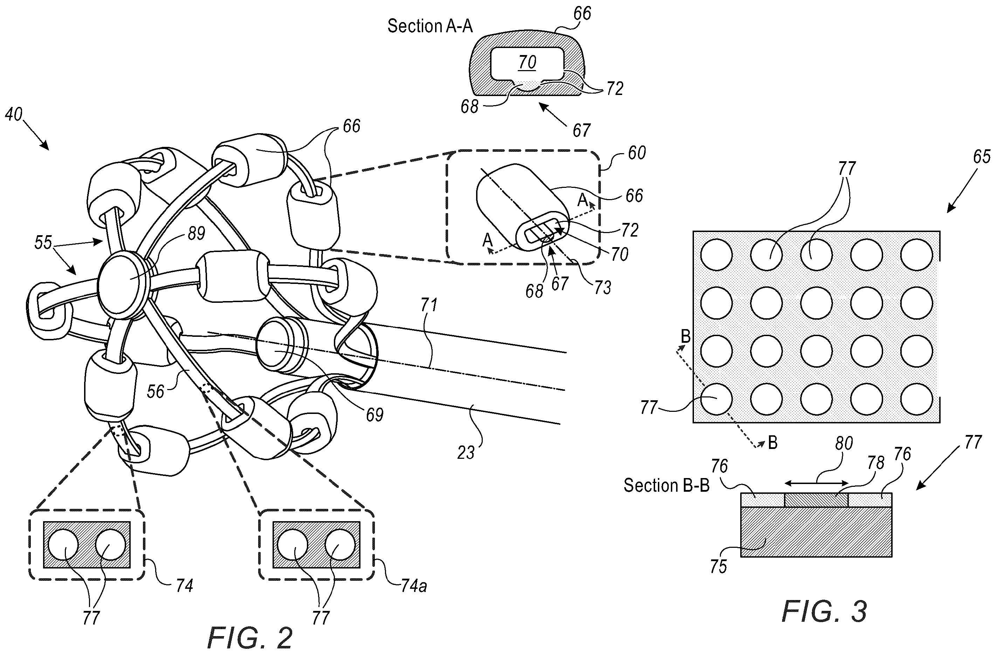

Reference is now made to an inset 25. In some examples, catheter 22 comprises a distal-end assembly 40 having multiple splines (shown in detail in Fig. 2 below), and a shaft 23 for inserting distal-end assembly 40 to a target location for ablating tissue in heart 26. During an Electrophysiology (EP) mapping and/or ablation procedure, physician 30 inserts catheter 22 through the vasculature system of a patient 28 lying on a table 29. Physician 30 moves distal-end assembly 40 to the target location in heart 26 using a manipulator 32 near a proximal end of catheter 22, which is connected to interface circuitry of processor 42.

In some examples, catheter 22 comprises a position sensor 39 of a position tracking system, which is coupled to the distal end of catheter 22, e.g., in close proximity to distal-end assembly 40. In the present example, position sensor 39 comprises a magnetic position sensor, but in other examples, any other suitable type of position sensor (e.g., other than magnetic based) may be used.

Reference is now made back to the general view of Fig. 1. In some examples, during the navigation of distal-end assembly 40 in heart 26, processor 42 receives signals from magnetic position sensor 39 in response to magnetic fields from external field generators 36, for example, for the purpose of measuring the position of distal-end assembly 40 in heart 26. In some examples, console 24 comprises a driver circuit 34, configured to drive magnetic field generators 36. Magnetic field generators 36 are placed at known positions external to patient 28, e.g., below table 29.

In some examples, processor 42 is configured to display, e.g., on a display 46 of console 24, the tracked position of distal-end assembly 40 overlaid on an image 44 of heart 26.

The method of position sensing using external magnetic fields is implemented in various medical applications, for example, in the CARTO^{™} system, produced by Biosense Webster Inc. (Irvine, Calif.) and is described in detail in U.S. Patents 5,391,199, 6,690,963, 6,484,118, 6,239,724, 6,618,612 and 6,332,089, in PCT Patent Publication WO 96/05768, and in U.S. Patent Application Publications 2002/0065455 A1, 2003/0120150 A1 and 2004/0068178 A1.

### DISTAL-END ASSEMBLY HAVING ABLATION ELECTRODES SENSING ELECTRODES AND TEMPERATURE SENSORS

Fig. 2 is a schematic, pictorial illustration of distal-end assembly 40 of catheter 22 in an expanded position, in accordance with examples of the present disclosure. In some examples, physician 30 may collapse and expand distal-end assembly 40 using manipulator 32, as will be described below.

In some examples, distal-end assembly 40 comprises an expandable basket catheter, which is coupled between shaft 23 and an apex 89 of distal-end assembly 40. Note that apex 89 is the distal-most part of catheter 22 which is typically (but not necessarily) orthogonal to an axis 71 of shaft 23.

In the present example, distal-end assembly 40 comprises multiple splines 55. Each spline 55 is made from or comprises a flexible substrate 56, such as an alloy of nickel-titanium (e.g., nitinol), or from any other one or more suitable materials, such as a multi-layered flexible printed circuit board. Note that splines 55 must be sufficiently flexible to conform to the tissue in question when being placed in contact therewith.

In some examples, distal-end assembly 40 comprises one or more ablation electrodes 66, in the present example, one or more ablation electrodes 66 are assembled on at least a given spline 55, and typically on each spline 55 of distal-end assembly 40.

Reference is now made back to an inset 60, showing one implementation of ablation electrode 66 in accordance with examples of the present disclosure.

In some examples, ablation electrode 66 includes a through hole (TH) 70 formed along an axis 73 of ablation electrode 66 and therethrough, so that spline 55 could be threaded through TH 70 as shown in the general view of Fig. 2.

In some examples, ablation electrode 66 includes a slot 67 which is formed in an inner surface 72 of TH 70, and is configured to contain a temperature sensor, such as a thermocouple (TC) 68, or any suitable device other than a temperature sensor. Slot 67 may be formed on any interior surface 72 of TH 70 such as in the example shown in Fig. 2 where the slot 67 is on the side within the boundary defined by the basket. Alternatively, the preferred location of slot 67 may be on the side of electrode 66 that is intended to face the tissue in question of heart 26.

Reference is now made to a sectional view AA of ablation electrode 66. In some examples, inner surface 72 is shaped such that TC 68 fits into and fills slot 67 without protruding into the area of TH 70, which is configured to snuggly fit over spline 55. Note that the slot 67 can be formed on the surface opposite to where slot 67 is presently shown so that the TC 68 can be closer to the tissue surface on which electrode contact surface 66 may impinge.

In some examples, when ablation electrode 66 is placed in contact with a given tissue of heart 26 that is intended to be ablated, TC 68 is configured to produce a thermal signal indicative of the temperature of the given tissue. In such examples, spline 55 is configured to: (i) conduct ablation signal(s) produced in console 24 and applied to the given tissue via ablation electrode 66, and (ii) conduct the thermal signal from TC 68 to processor 42 for analyzing the temperature of the given tissue. Note that TC 68 is controlled by processor 42 and is configured to produce the thermal signal: (i) while applying the ablation signal(s) to the given tissue, and (ii) when the ablation signal(s) are not applied to the given tissue.

Reference is now made back to the general view of Fig. 2. In some examples, when physician 30 moves distal-end assembly 40 of catheter 22 to the target location in heart 26, distal-end assembly 40 is in a collapsed position with all splines 55 straightened. When distal-end assembly 40 is positioned at the target location in heart 26, physician 30 typically reduces the distance between a distal end 69 of shaft 23 and apex 89 (e.g., by pulling apex 89 toward shaft 23, or by pushing distal end 69 of shaft 23 toward apex 89, or using any other technique), so as to have distal-end assembly 40 at an expanded position with splines 55 bent as shown in the example of Fig. 2. In the present example, physician 30 may use manipulator 32 (shown in Fig. 1 above) for controlling the distance between apex 89 and distal end 69 of shaft 23, and therefore, the amount of expansion of distal-end assembly 40. Alternatively, splines 55 may be shape set so that their natural shape is of a roughly spherical basket which is allowed to collapse when put into a sheath (not shown).

In some examples, distal-end assembly 40 comprises various types of diagnostic electrodes, such as sensing electrodes 77, which are coupled to (or formed on or disposed on) spline 55 and are typically positioned between adjacent ablation electrodes 66, or between an ablation electrode 66 and another element (e.g., apex 89 and/or shaft 23) coupled to spline 55. Note that at each position, spline 55 may have one or more sensing electrodes 77.

In some examples, at least one of and typically all sensing electrodes 77 are configured to be coupled to spline 66. When placed in contact with tissue of heart 26, each sensing electrode 77 is configured to produce a sensing signal indicative of an electrocardiogram (ECG) signal sensed from the tissue.

In some examples, processor 42 is configured to produce an EA map of the tissue in question (and other sections of heart 26). Based on the EA map, physician 30 (and/or processor 42) may determine one or more locations for applying the ablation signals to the tissue. Note that in principle it is possible to insert into heart 26 an additional catheter having sensing electrodes, such as sensing electrodes 77, for performing the EP mapping before or while inserting an ablation electrode. However, insertion of an additional catheter may: (i) prolong the ablation procedure, (ii) increase the risk for a safety event due to insertion of two catheters, and (iii) increase the cost associated with using two catheters instead of a single catheter having both sensing electrodes and ablation electrodes.

Reference is now made to insets 74 and 74a showing two pairs of sensing electrodes 77 coupled to each spline 55 at a selected position. In the example of inset 74 two sensing electrodes 77 are positioned between adjacent ablation electrodes 66, and in the example of inset 74a two sensing electrodes 77 are positioned between an ablation electrode 66 and apex 89.

In other examples, any other suitable number of sensing electrodes 77 may be implemented at each suitable location along spline 55. For example, one sensing electrode 77 between ablation electrode 66 and apex 89 and two or more sensing electrodes 77 implemented between two adjacent ablation electrodes 66.

In some examples, each sensing electrode 77 includes a circular shape and a diameter of about 0.15 mm. In alternative examples, the size and shape of each sensing electrode 77 may vary based on the position within each spline, or between splines. For example, a first sensing electrode 77 may have a square shape having a width of about 0.14 mm, a second sensing electrode 77 may have a circular shape having a diameter of about 0.15 mm, and a third sensing electrode 77 may have a circular shape having a diameter of about 0.18 mm.

As used herein, the terms "about" or "approximately" for any numerical values or ranges indicate a suitable dimensional tolerance that allows the part or collection of components to function for its intended purpose as described herein.

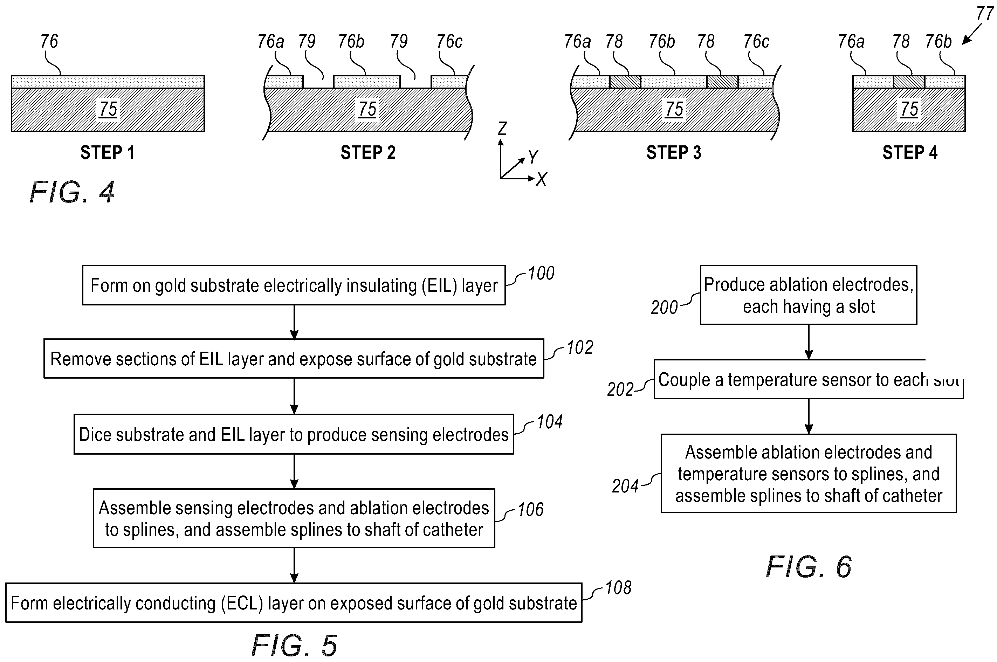

In some examples, each sensing electrode 77 includes an electrically conductive section and an electrically insulative section. Sensing electrodes 77 and their production processes are described in detail below in Figs. 3 and 4, respectively.

This particular configuration of distal-end assembly 40 is shown by way of example, in order to illustrate certain problems that are addressed by examples of the present disclosure and to demonstrate the application of these examples in enhancing the performance of such a catheterization and tissue sensing and ablation system. Examples of the present disclosure, however, are by no means limited to this specific sort of example distal end assembly, and the principles described herein may similarly be applied to other sorts of distal end assemblies and/or catheters used in tissue sensing, tissue ablation, a combination thereof and other medical applications applied to other organs of patient 28.

Note that by having ablation electrodes 66, TCs 68 and sensing electrodes 77 combined on distal-end assembly 40, physician 30 may insert distal-end assembly 40 into a cavity (e.g., right atrium or left atrium of heart 26), perform electro-anatomical mapping, and subsequently, perform the tissue ablation without retracting the catheter and/or inserting an additional catheter. In other words, the EA sensing, the temperature sensing, and the tissue ablation are carried out using one catheter having distal-end assembly 40 as shown in Fig. 2.

Additionally, or alternatively, sensing electrodes 77 may be implemented in distal end 40 using any other suitable technique. For example, using microelectrodes that are described in detail in U.S. Patent Applications 16/723,971 and 17/489,895 (which is a continuation-in-part of U.S. Patent Application 16/723,971). In some examples, distal-end assembly 40 may comprise these microelectrodes together with ablation electrodes 66 and TCs 68.

Fig. 3 is a schematic top view of multiple sensing electrodes 77 as produced, and a sectional view BB of one sensing electrode 77, in accordance with examples of the present disclosure.

In some examples, multiple sensing electrodes 77 are produced in one batch, e.g., in an array 65. Subsequently, sensing electrodes 77 are diced and separated into a single sensing sensor 77 or into a smaller array of multiple (e.g., a pair of) sensing sensors 77, as shown for example, in insets 74 and 74a of Fig. 2 above. The production process of sensing electrode(s) 77 is designed to reduce the manufacturing cost and is described in more detail in Fig. 4 below.

Reference is now made to sectional view BB of a selected sensing electrode 77. In some examples, each sensing electrode 77 comprises a gold substrate 75, which is formed on flexible substrate 56 of spline 55 and is configured to conduct the sensing signal(s) produced by the respective sensing electrode 77.

In some examples, sensing electrode 77 comprises a first polymer layer 76, which is formed over a first section of gold substrate 75 and is configured to electrically isolate between the tissue in question and gold substrate 75. In some examples, first polymer layer 76 may comprise an electrically insulating polymer, such as but not limited to polyethylene terephthalate (PET) or polyether block amide (PEBA).

In some examples, sensing electrode 77 comprises a second polymer layer 78, which is formed over a second section of gold substrate 75, and is configured to conduct the ECG signal between the tissue in question and gold substrate 75. Second polymer layer 78 is positioned between two sections of first polymer layer 76, i.e., the second section has a position different than that of the first section.

In some examples, second polymer layer 78 comprises an electrically conductive polymer, such as but not limited to poly(3,4-ethylenedioxythiophene) (PEDOT), poly(3, 4 ethylenedioxythiophene) polystyrene sulfonate (PEDOT:PSS), or any other suitable type of electrically-conductive polymer. In other examples, layer 78 may comprise non-polymer materials, such as electrochemically grown iridium oxide, electrochemically grown Titanium Nitride (TiN) or any other suitable type of electrically conductive alloy. In some examples, layer 78 is configured to reduce the impedance of electrode 77, and has a width 80, in the present example, a diameter of about 0.15 mm, as described in Fig. 2 above.

In some examples, the outer surface of polymer layer 78 is recessed relative to the outer surface of polymer layer 76. In such examples, when placed in contact with tissue, the outer surface of polymer layer 76 prevents direct contact with the interior of a sheath but allows for contact with the tissue, so as to prevent damage, such as erosion, to the surface of polymer layer 78.

In other examples, the outer surface of polymer layers 76 and 78 are flush with one another, so that when sensing electrode 77 is placed in contact with the tissue in question, both polymer layers 76 and 78 have a firmed contact with the tissue in question.

In alternative examples, the outer surface of polymer layer 78 slightly protrudes (e.g., about less than 5 µm) relative to the outer surface of polymer layer 76, so as to improve the sensing of the ECG signal by polymer layer 78.

### PRODUCING SENSING ELECTRODES INTENDED TO BE COUPLED TO DISTAL END ASSEMBLY

Fig. 4 is a block diagram that schematically illustrates a process flow for producing one of sensing electrodes 77 shown in Figs 2 and 3 above, in accordance with examples of the present disclosure.

At a step 1, polymer layer 76 is formed over the surface of gold substrate 75 such as by shrinking a polymer sleeve to provide a conformal surface. Note that the outer surface of polymer layer 76 is intended to be placed in contact with tissue, and polymer layer 76 is configured to electrically isolate between gold substrate 75 and the tissue in question.

At a step 2, a laser cutting system (not shown) is applied for cutting through polymer layer 76 and forming openings 79 at selected locations in which the second section of gold substrate 75 is exposed, as described for example in sectional view BB of Fig. 3 above. For the sake of conceptual clarity, polymer layer 76 has sections 76a, 76b and 76c that are surrounding openings 79. Note that sections 76a, 76b and 76c of polymer layer 76 are all made from the same layer and have the same size and shape.

In some examples, sections 76a, 76b and 76c and openings 79 represent a portion of array 65 shown in Fig. 3 above. Thus, the sectional view of Fig. 4 typically continues in an XY plane of an XYZ coordinate system of Fig. 4, and typically includes additional sections of polymer layer 76 and additional openings 79.

In other examples, openings 79 may be formed using any other suitable patterning and/or material removal process.

At a step 3, polymer layer 78 is applied into openings 79 (e.g., between sections 76a and 76b, and between sections 76b and 76c of polymer layer 76), and are disposed over the exposed surface of gold substrate 75. Polymer layer 78 is electrochemically grown by placing the catheter into a coating liquid and then running current between electrodes on the catheter to a counter electrode placed in the coating fluid. Coating in this manner allows polymer layer 78 to selectively be grown only on desired surfaces.

In some examples, the outer surface of polymer layer 78 is intended to be placed in contact with the tissue in question, and polymer layer 78 is configured to conduct the ECG signals between the tissue in question and gold substrate 75.

Note that in accordance with the examples shown in Fig. 3 above, steps 1-3 of the process flow of Fig. 4 are designed to produce multiple sensing electrodes 77 in one batch on gold substrate 75. At a step 4 that concludes the process flow of Fig. 4, sensing electrodes 77 are produced by dicing polymer layer 76 and gold substrate 75, and subsequently, by separating between adjacent sensing electrodes 77. In the example of step 4, section 76b of polymer layer 76 is cut-through or diced, together with gold substrate 75, along the Z-axis of the XYZ coordinate system of Fig. 4.

In some examples, the process flow may comprise additional steps after dicing and separating one or more sensing electrodes 77. For example, such processes may comprise polishing and preparing one or more surfaces are required for coupling each sensing electrode 77 to a respective spline 55 of distal-end assembly 40.

In alternative examples, step 3 may be carried out after step 4 and after: (i) assembling sensing electrodes 77 and ablation electrodes 66 to respective splines 55, and (ii) applying a curing step to distal-end assembly 40 for outgassing polymers of distal-end assembly 40, and (iii) preparing the surface of gold substrate 75 of opening 79 for electrochemically growing polymer layer 78. Note that for the electrochemical coating of polymer layer 78 the surface of gold substrate 75 of opening 79 must be clean because residues, and particularly non-conductive residues, may interfere with the electrochemical growing of polymer layer 78. Moreover, the one or more curing step(s) of distal-end assembly 40 may pollute the surface of sensing electrode 77 with outgassing polymers that may reduce the electrical conductivity, and therefore, the sensitivity of sensing electrode 77.

### PRODUCING CATHETER HAVING ABLATION ELECTRODES THERMOCOUPLES AND SENSING ELECTRODES

Fig. 5 is a flow chart that schematically illustrates a method for producing catheter 22 and distal-end assembly 40 having ablation electrodes 66 and the sensing electrodes 77 shown in Figs. 2-4 above, in accordance with an example of the present disclosure.

The method begins at an electrically insulating layer (EIL) formation step 100, with applying polymer layer 76 to the surface of gold substrate 75, as described in detail in step 1 of Fig. 4 above.

At an EIL patterning step 102, a laser cutting system is used for removing sections of polymer layer 76, and producing openings 79 for exposing the surface of gold substrate 75, as described in detail in step 2 of Fig. 4 above.

At a dicing and separation step 104, section 76b of polymer layer 76 and gold substrate 75 are diced or otherwise being cut along the Z-axis, and each sensing electrode 77 is produced, as described in detail in step 3 of Fig. 4 above.

At a catheter assembly step 106, sensing electrodes 77 and ablation electrodes 66 are coupled to splines 55. Moreover, splines 55 are assembled for producing distal-end assembly 40, and subsequently, (i) distal-end assembly 40 is coupled to shaft 23, and (ii) at least distal-end assembly 40 (and optionally shaft 23) undergo one or more curing processes for outgassing polymers away from catheter 22.

At an electrically conducting (ECL) layer formation step 108 that concludes the method, polymer layer 78 is applied into openings 79 and is formed over the exposed surface of section gold substrate 75, as described in detail in step 3 of Fig. 4 above. Note that the outer surface of polymer layer 78 is typically flush with that of polymer layer 76, so as to enable a firm contact between the tissue in question and polymer layer 78, and thereby, conduct the ECG signal(s) between the tissue and gold substrate 75.

Note that the electrochemical coating of polymer layer 78 is carried out on a completed device (e.g., distal-end assembly 40 or catheter 22), so as to ensure that the coating of polymer layer 78 is not polluted with outgassing polymers during the curing steps of distal-end assembly 40 and/or catheter 22.

The method of Fig. 5 is simplified for the sake of conceptual clarity, and the production method typically comprises additional steps, such as but not limited to coupling position sensor 39 to distal-end assembly 40 and the aforementioned curing of catheter 22 (the curing is carried out using any suitable process, such as but not limited to an ultraviolet (UV) curing process or a thermal curing process. In the UV curing process, part of, or all of the device is exposed to UV light (which may be monochromatic or broad spectrum, but typically includes a wavelength of about 405 nm or below) in order to cure adhesives. In the thermal curing process part of, or all of the device is placed within an oven (typically at a temperature between about 40°C and 95°C, but most commonly at a temperature of about 65°C) to cure the adhesives. Moreover, various types of connectors are coupled to the proximal end of catheter 22, e.g., for exchanging the sensing signals and ablation signals between the components of console 24 and distal-end assembly 40.

Fig. 6 is a flow chart that schematically illustrates a method for producing catheter 22 having ablation electrodes 66 and one or more TCs 68, in accordance with an example of the present disclosure.

The method begins at an ablation electrode production step 200, with producing ablation electrodes 66, at least one of and typically all ablation electrodes 66 have TH 70 for threading splines therethrough, and slot 67 for containing TC 68 or any other temperature sensor, as described in detail in Fig. 2 above.

At a temperature sensor coupling step 202, TC 68 is coupled to slot 67 of ablation electrode 66, as described in detail in Fig. 2 above.

At a catheter assembly step 204 that concludes the method, ablation electrodes 66 are coupled to splines 55, as described in detail in Fig. 2 above. Moreover, splines 55 are assembled for producing distal-end assembly 40, and subsequently, distal-end assembly 40 is coupled to shaft 23 for concluding the production of catheter 22.

Note that the electrochemical coating of polymer layer 78, which is described in detail in Fig. 5 above, is typically carried out on a completed device (e.g., distal-end assembly 40 or catheter 22), so as to ensure that the coating of polymer layer 78 is not polluted with outgassing polymers during the curing steps of distal-end assembly 40 and/or catheter 22.

These particular flow charts of Figs. 5 and 6 are provided by way of example, in order to illustrate certain problems in sensing electro-anatomical signals in tissue in question, and in applying ablation signals to tissue intended to be ablated. These problems are addressed by examples of the present disclosure and are intended to demonstrate the application of these examples in enhancing the performance of the sensing and ablation system of Fig. 1 above. Examples of the present disclosure, however, are by no means limited to these specific sorts of methods and/or to this specific sort of example system, and the principles described herein may similarly be applied to other sorts of tissue sensing and/or ablating system.

In other examples, the methods of Figs. 5 and 6 may be merged into a single method for producing distal-end assembly 40 having a combination of suitable ablation electrodes (such as one or more ablation electrodes 66), temperature sensors (such as one or more TCs 68) and sensing electrodes (such as one or more sensing electrodes 77) that are all incorporated on distal-end assembly 40.

Although the examples described herein mainly address basket catheters, or other sort of expandable catheters, used for cardiac ablation. The methods and systems described herein can also be used in other applications, such as in neurology, otolaryngology, and renal denervation.

It will thus be appreciated that the examples described above are cited by way of example, and that the present disclosure is not limited to what has been particularly shown and described hereinabove. Rather, the scope of the present disclosure includes both combinations and sub-combinations of the various features described hereinabove, as well as variations and modifications thereof which would occur to persons skilled in the art upon reading the foregoing description and which are not disclosed in the prior art. The invention is defined by the appended claims.

## Claims

1. A catheter (22), comprising:
a shaft (23) for insertion into an organ (26) of a patient (28);
an expandable distal-end assembly (40), which is coupled to the shaft (23) and comprises multiple splines (55);
at least an ablation electrode (66), which is configured: (a) to be coupled to a spline of the splines (55), and (b) when placed in contact with tissue of the organ (26), to apply an ablation signal to the tissue, wherein the ablation electrode (66) includes a slot (67), and wherein the ablation electrode includes a through hole (TH) formed along an axis of the ablation electrode; and
a temperature sensor (68), which is contained within the slot (67) and is configured, when the ablation electrode (66) is placed in contact with the tissue, to produce a thermal signal indicative of a temperature of the tissue.

2. The catheter according to claim 1, wherein the temperature sensor comprises a thermocouple.

3. The catheter according to claim 1, wherein the ablation electrode includes a first electrical contact with the spline for applying the ablation signal to the tissue, and the temperature sensor includes a second electrical contact with the spline, different from the first electrical contact, for conducting the thermal signal to the spline.

4. The catheter according to any preceding claim, wherein the TH is configured to snuggly fit over the spline and to thread the spline therethrough for coupling the ablation electrode to the spline.

5. The catheter according to any preceding claim, wherein the slot is formed in an inner surface of the TH, and wherein the slot is shaped such that the temperature sensor fits into the slot without protruding into the TH.

6. The catheter according to claim 5, wherein the slot is configured to form an electrical contact between the temperature sensor and the spline for conducting the thermal signal.

7. The catheter according to any one of claims 1-3, and comprising at least a sensing electrode, which is configured: (a) to be coupled to the spline, and (b) when placed in contact with the tissue, to produce a sensing signal indicative of an electrocardiogram (ECG) signal sensed in the tissue, the sensing electrode comprising: (i) a gold substrate, which is formed on the spline and is configured to conduct the sensing signal, (ii) a first polymer layer, which is formed over a first section of the gold substrate and is configured to electrically isolate between the tissue and the gold substrate, and (iii) a second polymer layer, which is formed over a second section of the gold substrate, different from the first section, and is configured to conduct the ECG signal between the tissue and the gold substrate.

8. The catheter according to claim 7, wherein the ablation electrode is positioned on the spline at a first position, and the sensing electrode is positioned on the spline at a second position, different from the first position.

9. A method for producing a catheter (22) according to any of the preceding claims, the method comprising:
producing one or more ablation electrodes (66) for applying, to tissue of an organ (26), one or more ablation signals, respectively, when at least an ablation electrode of the ablation electrodes (66) is placed in contact with the tissue, wherein at least the ablation electrode includes a slot (67) and wherein producing the ablation electrode comprises forming a through hole (TH) along an axis of the ablation electrode;
coupling, within the slot (67), a temperature sensor (68) for producing a thermal signal indicative of a temperature of the tissue when the ablation electrode is placed in contact with the tissue;
coupling the one or more ablation electrodes (66) to at least a spline (55) of an expandable distal-end assembly (40), wherein when placed in contact with the organ (26), the spline (55) conforms to the tissue; and
coupling the expandable distal-end assembly (40) to a shaft for insertion into the organ.

10. The method according to claim 9, wherein coupling the temperature sensor comprises coupling a thermocouple within the slot.

11. The method according to claim 9, and comprising producing: (i) a first electrical contact between the ablation electrode and the spline for applying the ablation signal to the tissue, and (ii) a second electrical contact, different from the first electrical contact, for conducting the thermal signal between the temperature sensor and the spline.

12. The method according to any one of claims 9 - 11, wherein the TH is formed to snuggly fit over the spline and to thread the spline therethrough for coupling the ablation electrode to the spline.

13. The method according to any one of claims 9 - 11, wherein forming the slot comprises forming the slot in an inner surface of the TH, and wherein the slot is shaped such that the temperature sensor fits into the slot without protruding into the TH.

14. The method according to claim 14, wherein coupling the temperature sensor comprises forming an electrical contact between the temperature sensor and the spline for conducting the thermal signal.

15. The catheter according to any one of claims 1-3 or the method according to any one of claims 9-11, wherein the temperature sensor is configured to produce the thermal signal: (i) while the ablation electrode applies the ablation signal to the tissue, and (ii) when the ablation electrode is placed in contact with the tissue and does not apply the ablation signal to the tissue.

16. The method according to any one of claims 9-11, and comprising coupling to the spline at least a sensing electrode, which when placed in contact with the tissue, produces a sensing signal indicative of an electrocardiogram (ECG) signal sensed from the tissue, the sensing electrode comprising: (i) a gold substrate, which is formed on the spline for conducting the sensing signal, (ii) a first polymer layer, which is formed over a first section of the gold substrate and for electrically isolating between the tissue and the gold substrate, and (iii) a second polymer layer, which is formed over a second section of the gold substrate, different from the first section, for conducting the ECG signal between the tissue and the gold substrate.

17. The method according to claim 16, wherein coupling the one or more ablation electrodes and the sensing electrode comprises, positioning on the spline: (i) at least the ablation electrode at a first position, and (ii) the sensing electrode at a second position, different from the first position.

## Patentansprüche

1. Katheter (22), umfassend:
einen Schaft (23) für ein Einführen in ein Organ (26) eines Patienten (28);
eine expandierbare Distalendanordnung (40), die mit dem Schaft (23) gekoppelt ist und mehrere Keile (55) umfasst;
mindestens eine Ablationselektrode (66), die konfiguriert ist: (a) um an einen Keil der Keile (55) gekoppelt zu werden, und (b) wenn in Kontakt mit Gewebe des Organs (26) platziert, um ein Ablationssignal an das Gewebe anzulegen, wobei die Ablationselektrode (66) einen Schlitz (67) einschließt, und wobei die Ablationselektrode eine Durchgangsbohrung (TH) einschließt, die entlang einer Achse der Ablationselektrode ausgebildet ist; und
einen Temperatursensor (68), der innerhalb des Schlitzes (67) enthalten ist und, wenn die Ablationselektrode (66) in Kontakt mit dem Gewebe platziert ist, konfiguriert ist, um ein thermisches Signal zu erzeugen, das eine Temperatur des Gewebes anzeigt.

2. Katheter nach Anspruch 1, wobei der Temperatursensor ein Thermoelement umfasst.

3. Katheter nach Anspruch 1, wobei die Ablationselektrode einen ersten elektrischen Kontakt mit dem Keil zum Anlegen des Ablationssignals an das Gewebe einschließt, und der Temperatursensor einen zweiten elektrischen Kontakt mit dem Keil einschließt, der sich von dem ersten elektrischen Kontakt unterscheidet, zum Leiten des thermischen Signals zu dem Keil.

4. Katheter nach einem der vorstehenden Ansprüche, wobei die TH konfiguriert ist, um eng über den Keil zu passen und den Keil dahindurchzufädeln, zum Koppeln der Ablationselektrode mit dem Keil.

5. Katheter nach einem der vorstehenden Ansprüche, wobei der Schlitz in einer inneren Oberfläche der TH ausgebildet ist, und wobei der Schlitz derart geformt ist, dass der Temperatursensor in den Schlitz passt, ohne in die TH vorzustehen.

6. Katheter nach Anspruch 5, wobei der Schlitz konfiguriert ist, um einen elektrischen Kontakt zwischen dem Temperatursensor und dem Keil zum Leiten des thermischen Signals auszubilden.

7. Katheter nach einem der Ansprüche 1 bis 3, und umfassend mindestens eine Erfassungselektrode, die konfiguriert ist: (a) um an den Keil gekoppelt zu werden, und (b) wenn in Kontakt mit dem Gewebe platziert, um ein Erfassungssignal zu erzeugen, das ein Elektrokardiogrammsignal (EKG-Signal) anzeigt, das in dem Gewebe erfasst wird, die Erfassungselektrode umfassend:
(i) ein Goldsubstrat, das auf dem Keil ausgebildet ist und konfiguriert ist, um das Erfassungssignal zu leiten, (ii) eine erste Polymerschicht, die über einem ersten Abschnitt des Goldsubstrats ausgebildet ist und konfiguriert ist, um zwischen dem Gewebe und dem Goldsubstrat elektrisch zu isolieren, und (iii) eine zweite Polymerschicht, die über einem zweiten Abschnitt des Goldsubstrats, der sich von dem ersten Abschnitt unterscheidet, ausgebildet ist und konfiguriert ist, um das EKG-Signal zwischen dem Gewebe und dem Goldsubstrat zu leiten.

8. Katheter nach Anspruch 7, wobei die Ablationselektrode an dem Keil an einer ersten Position positioniert ist und die Erfassungselektrode an dem Keil an einer zweiten Position positioniert ist, die sich von der ersten Position unterscheidet.

9. Verfahren zum Erzeugen eines Katheters (22) nach einem der vorstehenden Ansprüche, das Verfahren umfassend:
Erzeugen einer oder mehrerer Ablationselektroden (66) zum Anlegen eines oder mehrerer Ablationssignale an Gewebe eines Organs (26), wenn mindestens eine Ablationselektrode der Ablationselektroden (66) in Kontakt mit dem Gewebe platziert wird, wobei mindestens die Ablationselektrode einen Schlitz (67) einschließt und wobei das Erzeugen der Ablationselektrode das Ausbilden einer Durchgangsbohrung (TH) entlang einer Achse der Ablationselektrode umfasst;
Koppeln, innerhalb des Schlitzes (67), eines Temperatursensors (68) zum Erzeugen eines thermischen Signals, das eine Temperatur des Gewebes anzeigt, wenn die Ablationselektrode in Kontakt mit dem Gewebe platziert wird;
Koppeln der einen oder der mehreren Ablationselektroden (66) an mindestens einen Keil (55) einer expandierbaren Distalendanordnung (40), wobei sich, wenn in Kontakt mit dem Organ (26) platziert, der Keil (55) an das Gewebe anpasst; und
Koppeln der expandierbaren Distalendanordnung (40) mit einem Schaft für das Einführen in das Organ.

10. Verfahren nach Anspruch 9, wobei das Koppeln des Temperatursensors das Koppeln eines Thermoelements innerhalb des Schlitzes umfasst.

11. Verfahren nach Anspruch 9, und umfassend das Erzeugen von: (i) einem ersten elektrischen Kontakt zwischen der Ablationselektrode und dem Keil zum Anlegen des Ablationssignals an das Gewebe, und (ii) einem zweiten elektrischen Kontakt, der sich von dem ersten elektrischen Kontakt unterscheidet, zum Leiten des thermischen Signals zwischen dem Temperatursensor und dem Keil.

12. Verfahren nach einem der Ansprüche 9 bis 11, wobei die TH ausgebildet ist, um eng über den Keil zu passen und den Keil dahindurchzufädeln, um die Ablationselektrode mit dem Keil zu koppeln.

13. Verfahren nach einem der Ansprüche 9 bis 11, wobei das Ausbilden des Schlitzes das Ausbilden des Schlitzes in einer inneren Oberfläche der TH umfasst, und wobei der Schlitz derart geformt ist, dass der Temperatursensor in den Schlitz passt, ohne in den TH vorzustehen.

14. Verfahren nach Anspruch 14, wobei das Koppeln des Temperatursensors das Ausbilden eines elektrischen Kontakts zwischen dem Temperatursensor und dem Keil zum Leiten des thermischen Signals umfasst.

15. Katheter nach einem der Ansprüche 1 bis 3 oder Verfahren nach einem der Ansprüche 9 bis 11, wobei der Temperatursensor konfiguriert ist, um das thermische Signal zu erzeugen: (i) während die Ablationselektrode das Ablationssignal an das Gewebe anlegt, und (ii) wenn die Ablationselektrode in Kontakt mit dem Gewebe platziert wird und das Ablationssignal nicht an das Gewebe anlegt.

16. Verfahren nach einem der Ansprüche 9 bis 11, und umfassend das Koppeln an den Keil mindestens einer Erfassungselektrode, die, wenn sie in Kontakt mit dem Gewebe platziert wird, ein Erfassungssignal erzeugt, das ein Elektrokardiogrammsignal (EKG-Signal) anzeigt, das aus dem Gewebe erfasst wird, die Erfassungselektrode umfassend: (i) ein Goldsubstrat, das auf dm Keil zum Leiten des Erfassungssignals ausgebildet ist, (ii) eine erste Polymerschicht, die über einem ersten Abschnitt des Goldsubstrats ausgebildet ist und zum elektrischen Isolieren zwischen dem Gewebe und dem Goldsubstrat dient, und (iii) eine zweite Polymerschicht, die über einem zweiten Abschnitt des Goldsubstrats, der sich von dem ersten Abschnitt unterscheidet, zum Leiten des EKG-Signals zwischen dem Gewebe und dem Goldsubstrat ausgebildet ist.

17. Verfahren nach Anspruch 16, wobei das Koppeln der einen oder der mehreren Ablationselektroden und der Erfassungselektrode umfasst, auf dem Keil zu positionieren: (i) mindestens die Ablationselektrode an einer ersten Position und (ii) die Erfassungselektrode an einer zweiten Position, die sich von der ersten Position unterscheidet.

## Revendications

1. Cathéter (22), comprenant :
une tige (23) pour insertion dans un organe (26) d'un patient (28) ;
un ensemble extrémité distale expansible (40), qui est accouplé à la tige (23) et comprend de multiples armatures (55) ;
au moins une électrode d'ablation (66), qui est configurée : (a) pour être accouplée à une armature des armatures (55), et (b) lorsqu'elle est placée en contact avec un tissu de l'organe (26), pour appliquer un signal d'ablation au tissu, dans lequel l'électrode d'ablation (66) comporte une fente (67), et dans lequel l'électrode d'ablation comporte un trou traversant (TH) formé le long d'un axe de l'électrode d'ablation ; et
un capteur de température (68), qui est contenu au sein de la fente (67) et est configuré, lorsque l'électrode d'ablation (66) est placée en contact avec le tissu, pour produire un signal thermique indiquant une température du tissu.

2. Cathéter selon la revendication 1, dans lequel le capteur de température comprend un thermocouple.

3. Cathéter selon la revendication 1, dans lequel l'électrode d'ablation comporte un premier contact électrique avec l'armature permettant d'appliquer le signal d'ablation au tissu, et le capteur de température comporte un second contact électrique avec l'armature, différent du premier contact électrique, permettant de conduire le signal thermique à l'armature.

4. Cathéter selon l'une quelconque revendication précédente, dans lequel le TH est conçu pour s'ajuster parfaitement par-dessus l'armature et pour faire passer l'armature à travers celui-ci pour un accouplement de l'électrode d'ablation à l'armature.

5. Cathéter selon l'une quelconque revendication précédente, dans lequel la fente est formée dans une surface interne du TH, et dans lequel la fente est mise en forme de telle sorte que le capteur de température s'ajuste dans la fente sans faire saillie dans le TH.

6. Cathéter selon la revendication 5, dans lequel la fente est conçue pour former un contact électrique entre le capteur de température et l'armature permettant de conduire le signal thermique.

7. Cathéter selon l'une quelconque des revendications 1 à 3, et comprenant au moins une électrode de captage, qui est configurée : (a) pour être accouplée à l'armature, et (b) lorsqu'elle est placée en contact avec le tissu, pour produire un signal de captage indiquant un signal d'électrocardiogramme (ECG) capté dans le tissu, l'électrode de captage comprenant :
(i) un substrat en or, qui est formé sur l'armature et est configuré pour conduire le signal de captage, (ii) une première couche polymère, qui est formée par-dessus une première section du substrat en or et est configurée pour isoler électriquement entre le tissu et le substrat en or, et (iii) une seconde couche polymère, qui est formée par-dessus une seconde section du substrat en or, différente de la première section, et est configurée pour conduire le signal ECG entre le tissu et le substrat en or.

8. Cathéter selon la revendication 7, dans lequel l'électrode d'ablation est positionnée sur l'armature au niveau d'une première position, et l'électrode de captage est positionnée sur l'armature au niveau d'une seconde position, différente de la première position.

9. Procédé permettant de produire un cathéter (22) selon l'une quelconque des revendications précédentes, le procédé comprenant :
la production d'une ou plusieurs électrodes d'ablation (66) permettant d'appliquer, au tissu d'un organe (26), un ou plusieurs signaux d'ablation, respectivement, lorsqu'au moins une électrode d'ablation des électrodes d'ablation (66) est placée en contact avec le tissu, dans lequel cette au moins une électrode d'ablation comporte une fente (67) et dans lequel la production de l'électrode d'ablation comprend la formation d'un trou traversant (TH) le long d'un axe de l'électrode d'ablation ;
l'accouplement, au sein de la fente (67), d'un capteur de température (68) permettant de produire un signal thermique indiquant une température du tissu lorsque l'électrode d'ablation est placée en contact avec le tissu ;
l'accouplement de la ou des électrodes d'ablation (66) à au moins une armature (55) d'un ensemble extrémité distale expansible (40), dans lequel lorsqu'elle est placée en contact avec l'organe (26), l'armature (55) épouse la forme du tissu ; et
l'accouplement de l'ensemble extrémité distale expansible (40) à une tige pour insertion dans l'organe.

10. Procédé selon la revendication 9, dans lequel l'accouplement du capteur de température comprend l'accouplement d'un thermocouple au sein de la fente.

11. Procédé selon la revendication 9, et comprenant la production : (i) d'un premier contact électrique entre l'électrode d'ablation et l'armature permettant d'appliquer le signal d'ablation au tissu, et (ii) d'un second contact électrique, différent du premier contact électrique, permettant de conduire le signal thermique entre le capteur de température et l'armature.

12. Procédé selon l'une quelconque des revendications 9 à 11, dans lequel le TH est formé pour s'ajuster parfaitement par-dessus l'armature et pour faire passer l'armature à travers celui-ci pour un accouplement de l'électrode d'ablation à l'armature.

13. Procédé selon l'une quelconque des revendications 9 à 11, dans lequel la formation de la fente comprend la formation de la fente dans une surface interne du TH, et dans lequel la fente est mise en forme de telle sorte que le capteur de température s'ajuste dans la fente sans faire saillie dans le TH.

14. Procédé selon la revendication 14, dans lequel l'accouplement du capteur de température comprend la formation d'un contact électrique entre le capteur de température et l'armature permettant de conduire le signal thermique.

15. Cathéter selon l'une quelconque des revendications 1 à 3 ou procédé selon l'une quelconque des revendications 9 à 11, dans lequel le capteur de température est configuré pour produire le signal thermique : (i) alors que l'électrode d'ablation applique le signal d'ablation au tissu, et (ii) lorsque l'électrode d'ablation est placée en contact avec le tissu et n'applique pas le signal d'ablation au tissu.

16. Procédé selon l'une quelconque des revendications 9 à 11, et comprenant l'accouplement à l'armature d'au moins une électrode de captage, qui lorsqu'elle est placée en contact avec le tissu, produit un signal de captage indiquant un signal d'électrocardiogramme (ECG) capté auprès du tissu, l'électrode de captage comprenant : (i) un substrat en or, qui est formé sur l'armature permettant de conduire le signal de captage, (ii) une première couche polymère, qui est formée par-dessus une première section du substrat en or et permettant d'isoler électriquement entre le tissu et le substrat en or, et (iii) une seconde couche polymère, qui est formée par-dessus une seconde section du substrat en or, différente de la première section, permettant de conduire le signal ECG entre le tissu et le substrat en or.

17. Procédé selon la revendication 16, dans lequel l'accouplement de la ou des électrodes d'ablation et de l'électrode de captage comprend, le positionnement sur l'armature : (i) d'au moins l'électrode d'ablation au niveau d'une première position, et (ii) de l'électrode de captage au niveau d'une seconde position, différente la première position.
